# EUROPEAN PATENT APPLICATION

(11) **EP 4 147 784 A1**
(43) Date of publication of application: **15.03.2023**
(21) Application number: 22194090.1
(22) Date of filing: 06.09.2022
(51) Int. Cl.: B03C 1/01, A61M 1/36, B03C 1/28

(54) **SYSTEMS AND METHODS FOR SEPARATING CELLS INCORPORATING A MAGNETIC SEPARATOR**

(30) Priority: 10.09.2021 US 202163242713 P
(71) Applicant: Fenwal, Inc., Lake Zurich, IL 60047 (US)
(72) Inventor: WEGENER, Christopher J., Lake Zurich, 60047 (US); THOMPSON, Kyle, Lake Zurich, 60047 (US)
(74) Representative: Maikowski & Ninnemann Patentanwälte Partnerschaft mbB

(57) **Abstract**

A method of operating a magnetic selector is provided. The magnetic selector includes a housing including a floor having a floor surface, a magnet carrier disposed on an opposite side of the floor from the floor surface and having at least one magnet disposed thereon, the magnet carrier being moveable relative to the floor between a first state wherein the magnet carrier is adjacent the floor and a second state wherein the magnet carrier is spaced from the floor. The method includes disposing a container on the floor surface, moving the magnet carrier to the first state to apply a magnetic field to a fluid in the container, moving the magnet carrier to the second state from the first state to disengage the magnetic field from the container, and removing the container.

## Description

### TECHNICAL FIELD

The present disclosure is generally directed to devices and methods of using a magnetic field. More particularly, the present disclosure is directed to systems and methods for applying a magnetic field to a fluid including cells, and methods for separating cells incorporating a magnetic selector.

### BACKGROUND

The processing of biological fluid such as blood or blood components typically involves using a reusable processing apparatus ("hardware") and a disposable fluid circuit adapted for mounting or other association with the reusable apparatus. The fluid circuit typically includes containers such as plastic bags and associated tubing that defines a flow path through the circuit. The disposable fluid circuit may also include one or more separation devices where the biological fluid/cells can be separated into two or more components, washed, or otherwise processed. Separation devices may separate the biological fluid based on centrifugal separation and/or, as described below, membrane separation. Other separation devices may use magnetic fields, for example, to select and/or separate target cells. Certain systems may use a combination of these separation devices, such as described in US Pub. Nos. 2017/0315121 and 2018/0172685. The present disclosure provides methods for separating cells incorporating a magnetic selector.

### SUMMARY

Systems and methods are disclosed for preparation of immunomagnetic selection and downstream wash and resuspension of a target immune-phenotypic cell subset from a heterogeneous cell starting or source material. A fluid processing system is provided, which utilizes a magnetic selector having an engageable magnetic array, and which may be automated. The system is capable of preparing apheresis collected (or other heterogeneous cell populations) material for immunomagnetic selection by automated washing incubation, and volume/concentration adjusting of the cellular material. The system also facilitates the removal of target /non-target cell fractions and subsequent washing/resuspension of the target/non-target fraction at desired cell concentrations for downstream use. The method is highly configurable to enable a user to define custom values and processing parameters to accommodate a large number of applications and procedures including different cell starting materials, different cell target/non-target cell populations, purity/recovery profiles, and magnetic reagents.

In a first aspect, a method of operating a magnetic selector is provided. The magnetic selector includes a housing including a floor having a floor surface, a magnet carrier disposed on an opposite side of the floor from the floor surface and having at least one magnet disposed thereon, the magnet carrier being moveable relative to the floor between a first state wherein the magnet carrier is adjacent the floor and a second state wherein the magnet carrier is spaced from the floor. The method includes disposing a container on the floor surface, moving the magnet carrier to the first state to apply a magnetic field to a fluid in the container, moving the magnet carrier to the second state from the first state to disengage the magnetic field from the container, and removing the container.

In a second aspect, a method of operating a biological fluid processing system incorporating a magnetic selector is provided. The biological fluid processing system includes a magnetic selector having a housing including a floor having a floor surface, a magnet carrier disposed on an opposite side of the floor from the floor surface of the selector and having at least one magnet disposed thereon, the magnet carrier being moveable relative to the floor between a first state wherein the magnet carrier is adjacent the floor and a second state wherein the magnet carrier is spaced from the floor. The method includes disposing a container on the floor surface, adding a fluid having magnetic particles to a fluid in the container to form a complex with target cells in the fluid, moving the magnet carrier to the first state to apply a magnetic field to the fluid in the container, moving the magnet carrier to the second state from the first state to disengage the magnetic field from the container, and removing the container.

In a third aspect, a method of operating a fluid processing system is provided. The fluid processing system includes a fluid processor that operates on a disposable fluid circuit that is connectable to a source container filled with a biological fluid and configured to separate the biological fluid from the source container into at least two volumes of material, a container connected to the fluid processor along a fluid pathway, a magnetic selector comprising a housing configured to receive the container and a magnet carrier, the housing including a floor having a floor surface upon which the container is disposed, and the magnet carrier being disposed in a space on an opposite side of the floor from the floor surface. The method includes pre-processing, processing, and post-processing states. The pre-processing state further includes selection of a procedure protocol, procedure setup, installation of the disposable fluid circuit on the fluid processor and attaching the source container and at least one solution container to the fluid processor. The processing state further includes introduction of the biologic fluid in the source container to the disposable fluid circuit, washing, harvesting and dilution of the of the biologic fluid, incubation with magnetic particles to perform negative selection incubation, moving the magnet carrier between a first state adjacent the floor to subject the container to a magnetic field and a second state spaced from the floor to remove the magnetic field, and removal of negative fraction comprising unbound cells that remain in suspension in the container. The post-processing state further comprising sealing the container and removing the final product, and removing the disposable fluid circuit.

### DESCRIPTION OF THE DRAWINGS

Fig. 1 is a perspective view of an embodiment of a magnetic selector, having a door in a closed state or position.
Fig. 2 is a perspective view of the magnetic selector of Fig. 1, with the door in an open state or position.
Fig. 3 is a partial cross-sectional view of the magnetic selector of Fig. 1, with the door in the open state and a magnet carrier spaced from a floor of the magnetic selector.
Fig. 4 is a partial cross-sectional view of the magnetic selector of Fig. 1, with the door in the closed state and the magnet carrier spaced from the floor of the magnetic selector.
Fig. 5 is a partial cross-sectional view of the magnetic selector of Fig. 1, with the door in the closed state and the magnet carrier adjacent the floor of the magnetic selector.
Fig. 6 is a perspective view of a fluid processing system incorporating the magnetic selector of Fig. 1, with a fluid circuit removed and the door of the magnetic selector in the open state or position.
Fig. 7 is a perspective view of the fluid processing system of Fig. 6, with the fluid circuit mounted and the door of the magnetic selector in the closed state or position.
Fig. 8 is a block diagram of a controller used in conjunction with the other elements of the system of Fig. 6.
Figs. 9A-9C are flow charts showing detailed steps of a method of operating the system of Fig. 6, and the magnetic selector incorporated therein.
Fig. 10 is a flow chart showing more general steps with respect to a method of operation of utilizing the magnetic selector described herein.

### DETAILED DESCRIPTION

A more detailed description of the devices and methods that utilize the devices in accordance with the present disclosure is set forth below. It should be understood that the description below of specific devices and methods is intended to be exemplary, and not exhaustive of all possible variations or applications. Thus, the scope of the disclosure is not intended to be limiting, and should be understood to encompass variations or embodiments that would occur to persons of ordinary skill in the art.

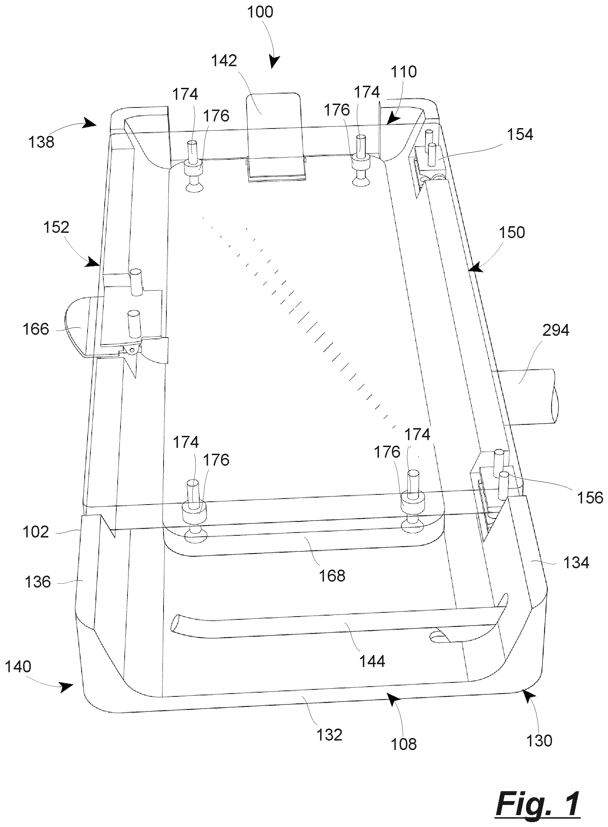

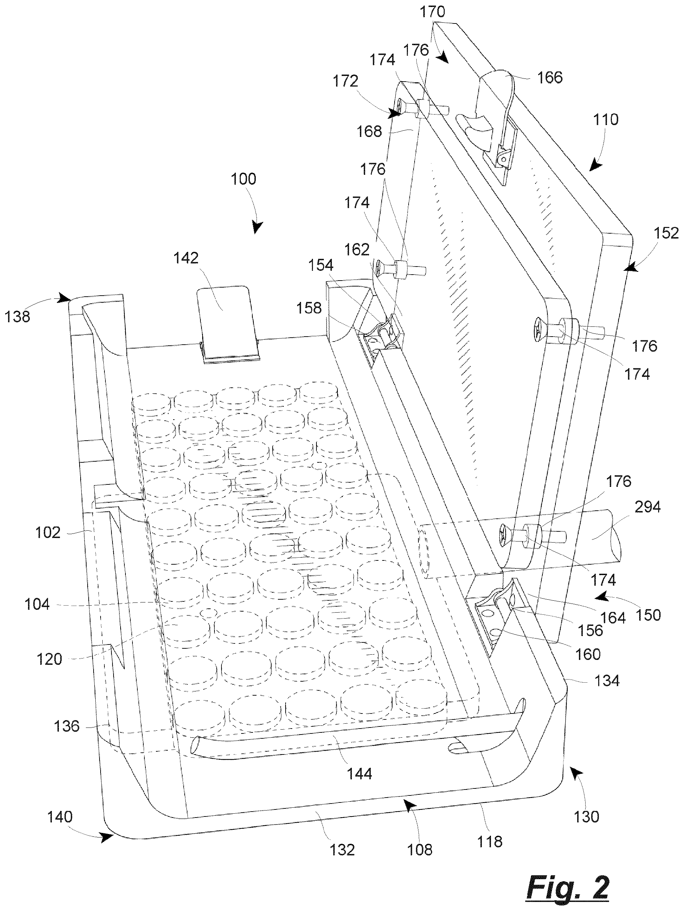

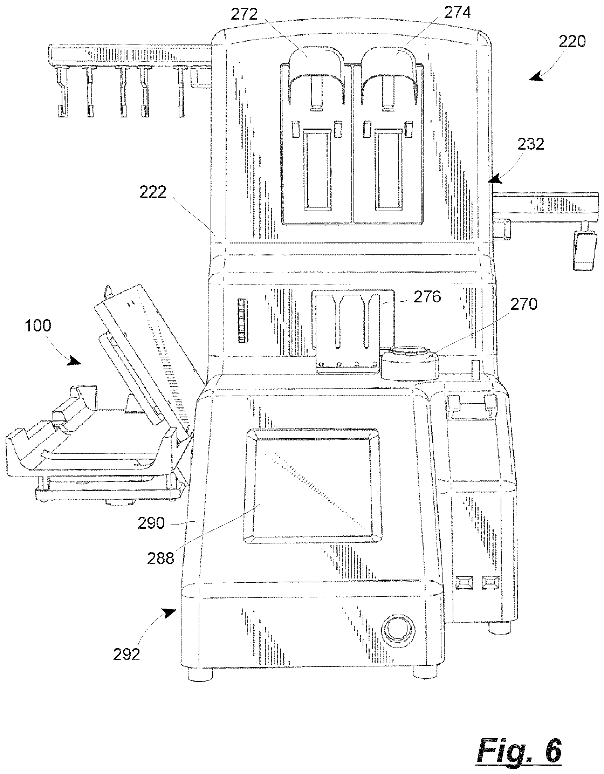

Turning first to Fig. 1, a magnetic selector 100 according to the illustrated embodiments includes a housing 102 (see, e.g., Figs. 1 and 2) and a magnet carrier 104 (see, e.g., Figs. 2-5). The magnetic selector 100 may also include a controller that is coupled (e.g., hardwired) to the magnet carrier 104 and that is configured to operate the magnet carrier 104 and other aspects of the magnetic selector 100. Alternatively, the magnetic selector 100 may be used with or incorporated into a fluid processing system, for example, as will be discussed below with reference to Figs. 6-8, which fluid processing system may include a controller that is configured to operate the magnet carrier 104 and the other aspects of the magnetic selector 100.

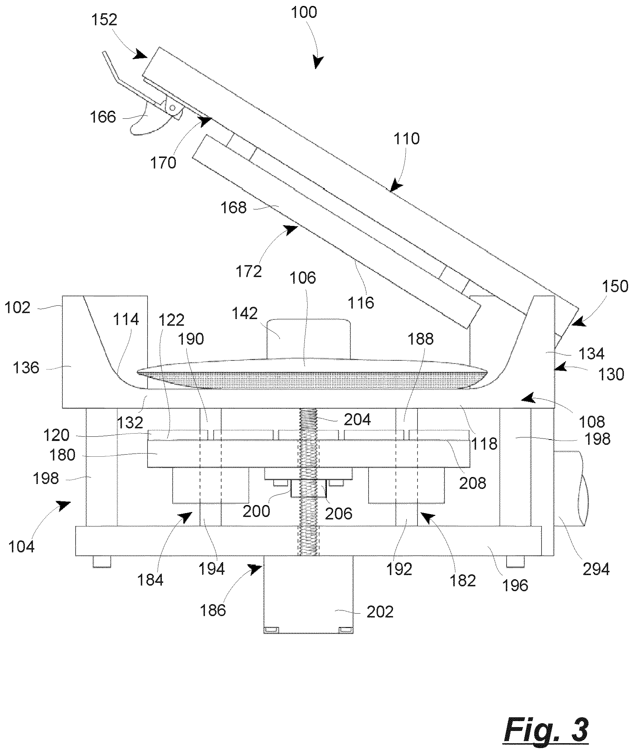

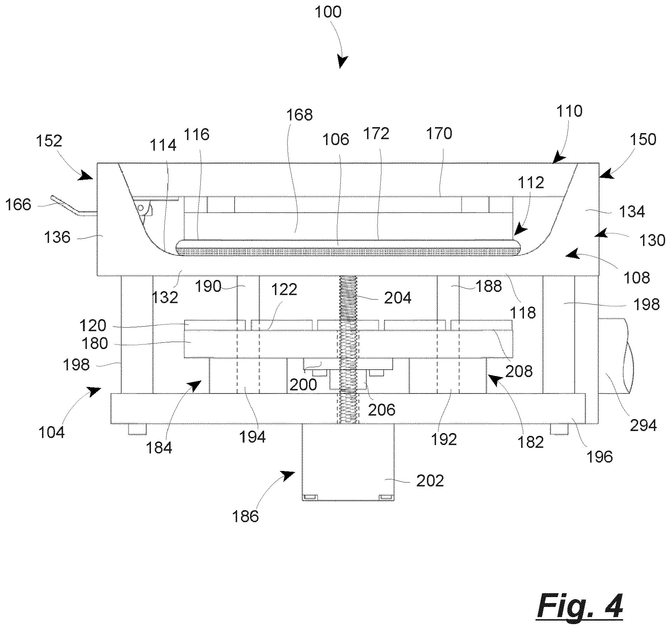

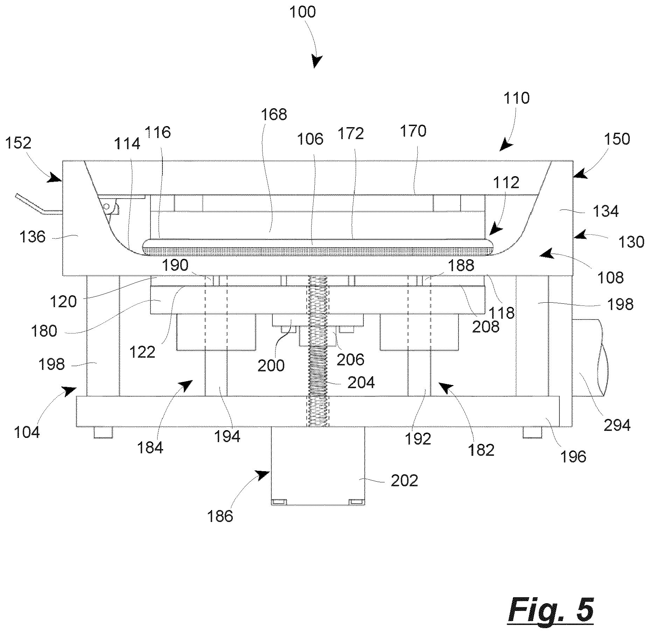

The housing 102 of the magnetic selector 100 is configured to receive a container 106 (Figs. 3-5), which may be flexible or rigid walled. As illustrated, the housing 102 includes a floor 108 upon which the container 106 is disposed. The housing may include a door 110 that is moveable relative to the floor 108. The door 110 is moveable (e.g., pivotable) between an open state (Figs. 2 and 3) and a closed state (Figs. 1, 4, and 5). The container 106 may be disposed in a space 112 (see, e.g., Figs. 4 and 5) between a floor surface 114 and a facing door surface 116 that are spaced a distance apart. As such, if the container 106 is flexible, it will assume a shape that is determined by the surfaces 114, 116, as if the container were a container of fixed or rigid shape instead. Alternatively, the container may be a rigid-walled container, with a shape selected to conform to the floor surface 114 and may maintain a shape that otherwise would be imposed by the door surface 116.

In the present example, the magnet carrier 104 is disposed on an opposite side 118 of the floor 108 from the container 106. See Figs. 2-5. The magnet carrier 104 has at least one magnet 120 disposed thereon. As is illustrated herein, the at least one magnet 120 may include a plurality of magnets (e.g., permanent magnets) disposed in a two-dimensional or three-dimensional array on a surface 122 of the magnet carrier 104. The magnet carrier 104 is moveable relative to the floor 108 between a first state (Fig. 5) wherein the magnet carrier 104 is adjacent (in close proximity to) the floor 108 and a second state (Figs. 3 and 4) wherein the magnet carrier 104 is spaced from the floor 108.

The magnetic selector 100 may be used in a number of immunomagnetic procedures. In general terms, these procedures may include disposing a container on the floor 108 (Fig. 3). If the container is of rigid construction, a door is not required. The procedure may require moving the magnet carrier 104 to the first state to apply a magnetic field to the fluid in the container (Fig. 5), moving the magnet carrier 102 to the second state from the first state to disengage the magnetic field from the container (Fig. 4), and removing the container (Fig. 3).

The magnetic selector 100 also may be used with a flexible container 106 for similar procedures, as more particularly shown in the Figures. Thus, the procedures may include disposing the flexible container 106 on the floor 108 (Fig. 3), moving the door 110 from the open state to the closed state with the flexible container 106 between the floor surface 114 and the facing door surface 116 (Fig. 4), moving the magnet carrier 104 to the first state to apply a magnetic field to the fluid in the container 106 (Fig. 5), moving the magnet carrier 102 to the second state from the first state to disengage the magnetic field from the container 106 (Fig. 4), moving the door 104 to the open state and removing the flexible container 106 (Fig. 3).

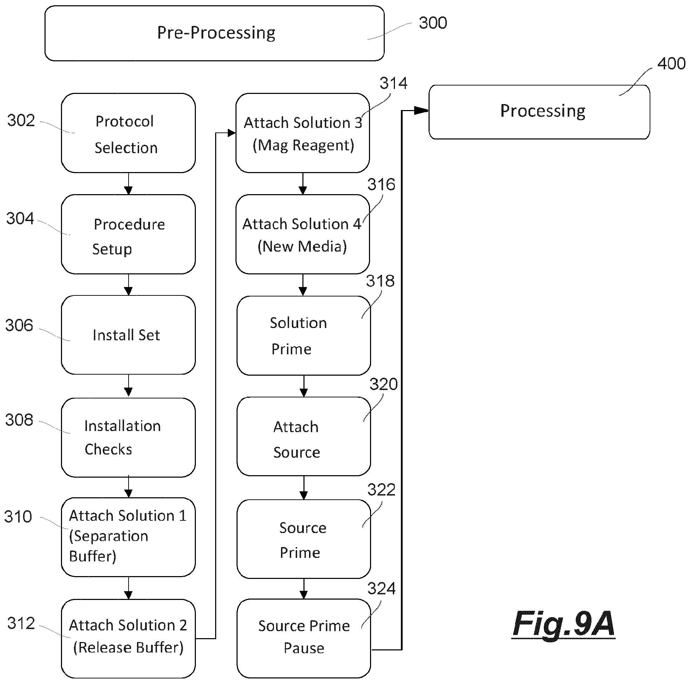

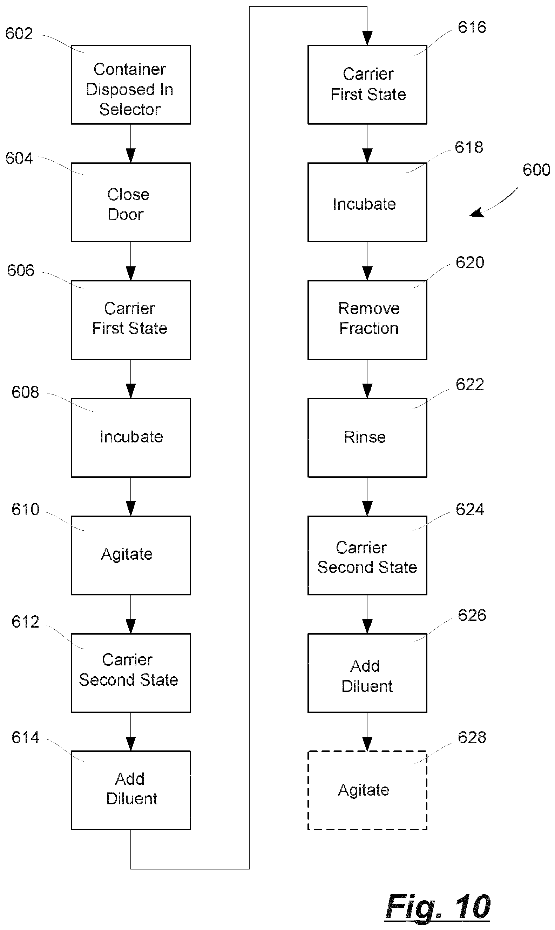

These procedures, whether using a rigid or flexible container, may be represented as a subset of a larger procedure, as shown and described with respect to the flow charts in Figs. 9A-9C and 10. The procedures also may include agitating the fluid in the container at specific steps in the procedure. For example, the procedures may include disposing the container 106 on the floor 108 within the magnetic selector 100. The door 110 may be moved to a closed state agitating the fluid in the container 106 with the container on the floor 108 after moving the magnet carrier 104 to the second state. The procedures may also include subsequently moving the magnet carrier 104 to the first state to apply a magnetic field to the fluid in the container 106 after agitating the fluid in the container 106, moving the magnet carrier 104 to the second state from the first state to disengage the magnetic field from the container 106, and adding diluent to the container 106 after moving the magnet carrier 104 to the second state. The procedures may also include moving the magnet carrier 104 to the first state to apply a magnetic field to the fluid in the container 106 after adding the diluent to the container 106, and then removing a portion of the fluid and diluent from the container 106. These actions may then be followed by adding diluent to the container 106 after removing a portion of the fluid and diluent from the container 106.

The procedures may also include, as a preliminary action, adding a magnetic particle or fluid containing magnetic particles to the fluid in the container 106 prior to moving the magnet carrier 104 to the first state to apply a magnetic field to the fluid in the container 106, the magnetic particle forming a complex with a target cell in the fluid. In fact, this preliminary action may be performed before the container 106 is disposed on the floor 108, although according to other embodiments, the magnetic particle may be added while the container 106 is disposed on the floor 108. Moreover, the magnetic particle may be attached to an intermediary, with the intermediary attaching to the target cell to form the complex, or the magnetic particle and the intermediary may be added to the fluid and the magnetic particle then attaches to the intermediary and the intermediary attaches to the target cell to form the complex.

The advantages of the magnetic selector 100 may include one or more of the following. Even though the container 106 may be a flexible container, by disposing the container 106 in the housing 104 with selected spacing between the floor 108 and door 110 (or the surfaces of the floor 108 and door 110), the container 106 assumes a rigid shape, permitting a more uniform fluid-gas (e.g., air) interface to be formed within the container 106 (see, e.g., Figs. 4 and 5). This more uniform interface is of assistance where, as here, the distance of the fluid from the magnet may affect the application of the magnetic field on the fluid, and in particular on the cells within the fluid. Similar advantages may be obtained where the container is a rigid-walled container. In addition, by providing a moveable magnet carrier 104, a complicated procedure involving applying and removing magnetic fields may be automated, permitting the procedure to be carried out in a single container, as opposed to moving the fluid between containers for example, although movement of the fluids between containers is possible as well. Further, by providing the moveable magnet carrier 104 on the side opposite the container 106, the structure and operation of the magnet carrier 104 may be simplified, as opposed to one which also must accommodate for the introduction and removal of the container 106 from the housing 102 (see, e.g., Fig. 3).

Having thus described the magnetic selector 100 in general terms, as to its structure and operation, the structure and operation of the magnetic selector 100 is now described in detail below. Further, the structure and operation of the magnetic selector 100 is described as incorporated into a fluid processing system, and in particular a cell processing system.

Starting first with Figs. 1 and 2, the magnetic selector 100 of the illustrated embodiment has a housing 102 including a tray 130. The tray 130 has a bottom wall 132 that defines the floor 108, and side walls 134, 136 that depend upward from the bottom wall 132/floor 108. As illustrated, the tray 130 substantially lacks walls at either end 138, 140 (e.g., to facilitate introduction and removal of the container 106 and structures attached to the container 106), although the tray 130 may be provided with equipment at either end 138, 140 to secure the container 106 within the housing 102. For example, a clip 142 may be attached to the floor 108 at the end 138, which clip 142 may include one or more spring loaded jaws that receive an end of the container 106 therebetween to secure an end of the container 106 to the housing 102. At the other end 140 of the tray 130, a bar 144 may be attached to the floor 108 under which an opposite end of the container 106 may be passed, which end of the container 106 may have other structures (e.g., ports, such as may be configured for sampling or for access to a fluid circuit) may be disposed. According to other embodiments, the tray 130 may also have end walls at the ends 138, 140.

As will be seen in Figs. 3-5, the side walls 134, 136 may be spaced from each other such that the container 106 is received therebetween without touching one or both the side walls 134, 136. This spacing of the side walls 134, 136, permits the container 106 to be disposed between the floor 108 and the door 110 into a more uniform shape (compare Fig. 3 with Figs. 4 and 5). While the amount of change in the shape of the flexible container 106 may be less than that illustrated in Figs. 3-5, one will recognize that the distances between various points within the container 106, and in particular along a fluid-gas (e.g., air) interface within the container 106, will be more uniform when the door 110 is moved into its closed state (e.g., Figs. 4 and 5). As mentioned above, the container may alternatively be a rigid-walled container, in which case the door 110 may be optional because the advantages of having the container conform to the tray 130 and door 110 may be achieved through the rigid wall construction of the container.

The door 110 is pivotally mounted along one edge 150 to a first of the side walls (e.g., side wall 134) and has an opposing edge 152 abutting a second of the side walls (e.g., side wall 136) opposite the first of the side walls (134) in the closed state (see Figs. 1, 4, and 5). As best seen In Figs. 1 and 2, the housing 102 may include one or more hinges 154, 156 that are attached at a first end 158, 160 to the side wall 134 and that are attached a second end 162, 164 to the door 110. This permits the door 110 to pivot or rotate between the open state (e.g., Fig. 2) and the closed state (Fig. 1). The door 110 may also include a latch 166 disposed along the edge 152, which latch 166 may cooperate with the side wall 136 to maintain the door 110 in the closed state.

There is a distance between the floor surface 114 and the facing door surface 116 that is determined at least in part by a height of the first and second side walls 134, 136. As illustrated in the Figures, and in particular Figs. 3-5, the portion of the surfaces 114, 116 abutting the container 106 are flat and parallel such that the fixed distance is uniform between those surfaces 134, 136 from side wall 134 to side wall 136 and from end 138 to end 140. This arrangement provides for a fluid-gas interface within the container 106 that is a fixed distance from the floor surface 114 that is uniform from side wall 134 to side wall 136 and from end 138 to end 140. It will be recognized that changes in the surfaces 114, 116 could provide a fixed distance, but one that is not uniform between those surfaces 114, 116 from side wall 134 to side wall 136 and from end 138 to end 140. As such, the flexible container 106 would still be forced into a shape like a rigid container, but with different distances relative to the floor surface 114, which might be desirable with a different arrangement of magnets than is illustrated herein, for example.

As is also illustrated in the Figures, the door 110 may include a spacer 168 disposed on a surface 170 of the door 110 facing the floor surface 114 with the door 110 in the closed state (see, e.g., Fig. 4). The spacer 168 has a spacer surface 172 that faces the floor surface 114 with the door 110 in the closed state, the facing door surface 116 including the spacer surface 172. As illustrated in the Figures, the spacer surface 172 is coextensive with the facing door surface 116. It will be recognized that according to other embodiments, the spacer 168 may not be included, in which case the surface 170 may be coextensive with the facing door surface 116.

The spacer 168 may be removeable, such that the spacer 168 may be included or not, to accommodate containers 106 of differing sizes, for example. The spacer 168 may also be adjustable, such that the fixed distance between the floor surface 114 and door surface 116 may be changed. For example, as illustrated, the spacer 168 is attached to the surface 170 of the door 110 through the use of four fasteners 174 located at the corners of the spacer 168. As can be seen, for example in Fig. 2, the spacer 168 does not abut the surface 170 (although it could according to other embodiments), but a post 176 is disposed between the corners of the spacer 168 and the surface 170. The height of the post 176 determines in part (along with the thickness of the spacer 168, for example) the distance between the surfaces 114, 116. By removing a post of a first height and replacing it with a post of a second height, the distance between the surfaces 114, 116 may be changed. This adjustment also may permit accommodation of containers 106 of differing sizes.

It will be further recognized that the adjustment of the spacer 168 to vary the distances 114, 116 may be provided by other mechanisms, such as an adjustable screw at each of the corners of the spacer 168, for example. The adjustment may even be automated according to certain embodiments, wherein a motor is used to operate the adjustable screw or screws, a fashion similar to that of the linear actuator described below in regard to the magnet carrier 104.

Moving to Figs. 3-5, the magnet carrier 104 may include a plate 180 on which the at least one magnet 120 is disposed. The plate 180 is translatably attached to the floor 108 to translate between a first state (Fig. 5) and the second state (Figs. 3 and 4). As mentioned previously, the magnet carrier 104 may be coupled to a controller, which controller automatically translates the plate 180 between the first and second states according to a procedure (e.g., a procedure programmed into the controller).

As is illustrated, the magnet carrier 104 includes a plurality of linear bearings 182, 184 and a linear actuator 186. The linear bearings 182, 184 are attached at one end 188, 190 to the floor 108, and the plate 180 is mounted on the linear bearings 182, 184. As is illustrated in Figs. 3-5, the other end 192, 194 of the bearings 182, 184 is attached to a support plate 196 that is secured to the floor 108 by a plurality of posts 198. The linear actuator 186 is attached to the plate 180 at 200, and the linear actuator 186 is configured to move the plate 180 along the linear bearings 182, 184 between the first state and the second state. As is illustrated in Figs. 3-5, the linear actuator 186 may include a motor 202 that is mounted on the support plate 196, and that rotates a lead screw 204 that is received in a nut 206 secured to the plate 180, the rotation of the lead screw 204 and its cooperation with the nut 206 causing the plate 180 to move between the first and second states, or positions.

As was mentioned above, the at least one magnet 120 may include a plurality of magnets, as illustrated in Figs. 2-5. The array 120 may be two-dimensional where a surface 208 of the plate 180 is flat, or may be three-dimensional where the surface 208 of the plate 180 is not flat or where the individual magnets are mounted on posts of varying heights, for example. According to the illustrated embodiments, the at least one magnet 120 may be a permanent magnet, although according to other embodiments the magnets 120 may be electromagnets instead.

In operation, the operator, also referred to as the user, may open the door 110 by first opening the latch 166 and rotating the door 110 about its hinges 154, 156. The user may then dispose the flexible container 106 on the floor 108 (Fig. 3), and secure the container 106 in place using the clip 142 and bar 144. The user may then move the door 110 from the open state to the closed state, with the flexible container 106 between the floor surface 114 and the facing door surface 116 (e.g., Fig. 4). At this time, the magnet carrier 104 may be in the second state as illustrated.

The magnet carrier 104 then may be moved, for example automatically by an electronic controller, to the first state to apply a magnetic field to the fluid in the container 106 (Fig. 5). This may be performed by causing the motor 202 to rotate the lead screw 204 in a first direction. The magnet carrier 104 may remain in the first state for a period of time. The magnet carrier 104 may then be moved to return to the second state from the first state to disengage the magnetic field from the container 106 (Fig. 4), after which the user may move the door 110 to the open state and remove the flexible container 106 (Fig. 3).

It will be understood that other steps may precede those mentioned in the preceding two paragraphs. Further, other steps may be performed between those states described. Finally, other steps may occur after the states described above.

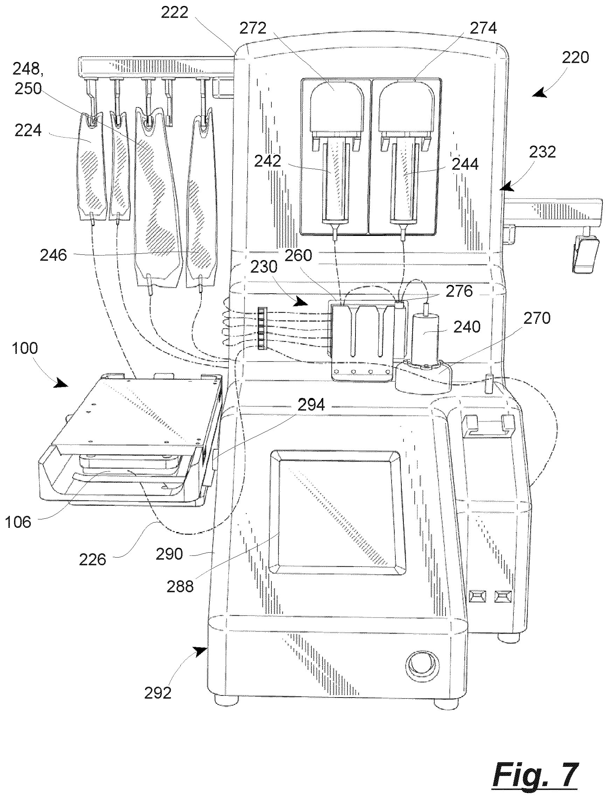

While it is possible to use the magnetic selector independently, it is also possible to use the selector in combination with or as incorporated into a fluid processing system, such as a cell processing system. For example, Figs. 6 and 7 illustrate the selector 100 as incorporated into a fluid processing system, such as the processing system disclosed in US Pub. No. 2021/0046426, which reference is incorporated herein in its entirety. It is also possible too that the magnetic separator 100 may be used with other processing systems, such as are described in US Pub. Nos. 2017/0315121 and 2018/0172685 in combination with other magnetic separators or selectors.

According to such an embodiment, as may be seen in Fig. 7, a fluid processing system 220 includes a fluid processor 222 connectable to a source container 224 filled with a biological fluid, and configured to separate the biological fluid from the source container 224 into at least two streams of material. "Biological fluid" includes without limitation blood and blood components, and "cell" or "biological cell" includes without limitation blood cells, such as red cells, white cells, and platelets. The processor 222 is connected to the container 106 disposed in the magnetic selector 100 along a fluid pathway 226.

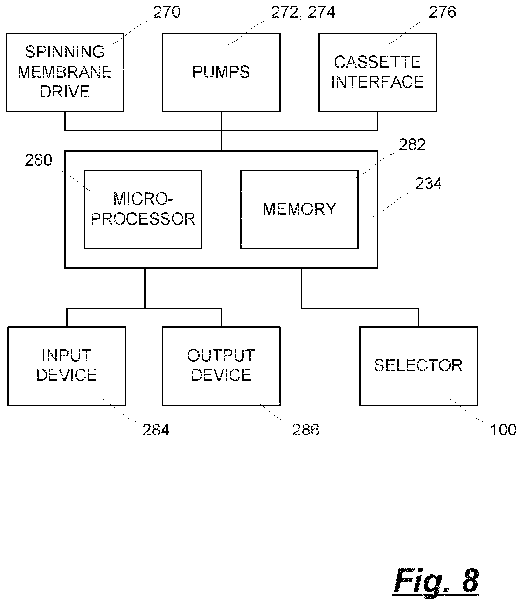

The processor 222 includes a disposable fluid circuit (also referred to as a set or kit) 230 used in combination with reusable processing machine, or hardware 232. The circuit 230 may be a "closed" system or circuit, in which the interior of the system, e.g., the flow paths, container, etc., are not exposed or "opened" to the outside environment; the circuit 230 may be referred to as closed even where additional containers are attached to the circuit 230, for example before or during a procedure. A control unit (or controller) 234 (see Fig. 8) is coupled to the processor 222, including the circuit 230 and hardware 232. The controller 234 is configured to operate the processor 230, and hardware 232 according to a procedure or process to produce or generate a product in combination with the selector 100. For example, the magnetic selector 100 may be included as part of the system 220 to provide an additional selection of cells of interest or target cells, which target cells were initially separated from a biological fluid by the processor 230, 232.

As seen in Figs. 6 and 7, the disposable fluid circuit 230 is connectable to the source container 224 of fluid, in particular biological fluid. The disposable fluid circuit 230 includes a spinning membrane separator 240 that is used to process the fluid received from the source container 224. The flow of fluid from the source container 224, through the spinning membrane separator 240, and to the one or more containers, such as the container 106, is achieved through the use of first and second syringes 242, 244, which are in fluid communication with the source container 224, the spinning membrane separator (or spinning membrane for short) 240, and the container 106. The syringes 242, 244 also may be in fluid communication with a number of other containers 246, 248, 250.

The flow of the fluid between the containers 224, 106, 246, 248, 250, the spinning membrane 240, and the syringes 242, 244 is controlled using a flow control cassette 260, which cassette 260 may be connected to each of the foregoing by tubing, or lines. In addition, the cassette 260 may include internal flow paths that are defined in part by a plurality of separate channels or passages, which in turn may be contained within and may be defined by the structure (e.g., housing) of the cassette 260. The channels may be connected at a plurality of selectable junctions, which may control the flow of fluid from one channel to another. These selectable junctions may also be referred to as valves, valve stations, or clamps, because, as illustrated, the selectable junctions provide controlled access between the channels. The cassette 260 may also include sensor stations, by which sensors may be associated with the flow paths within the cassette 260 to determine characteristics of the flow therein, such as pressure. Preferably, the length of each of the lines and channels is kept as short as possible to further minimize the internal volume of the fluid circuit 230.

As illustrated in Fig. 7, the spinning membrane 240 and the syringes 242, 244 may be integrally formed as part of (i.e., as one piece with) the cassette 260, to further reduce the tubing volume associated with the kit 230. According to other embodiments, the spinning membrane 240 and/or the syringes 242, 244 may be attached to the remainder of the fluid circuit 230 at the time of use, as may be the case with one or more of the containers 224, 106, 246, 248, 250. Again, as illustrated in Fig. 7, the container 246 and container 106 may be integrally formed with the cassette 260.

As seen in Figs. 6-8, the reusable hardware component (or reusable hardware for short) 232 includes a drive 270 for the spinning membrane separator 240, a syringe pump 272, 274 for each respective syringe 242, 244, and a control cassette interface 276 that is associated with the flow control cassette 260 when the fluid circuit 230 is disposed on the hardware 232 (e.g., is mounted on the hardware 232). The cassette interface 276 includes actuators and sensors that are associated with the clamps and sensor stations of the flow control cassette 260, and are configured to operate the clamps or sense characteristics of the fluid, respectively.

The reusable hardware 232 is coupled to the controller 234 which is configured to control operation of the system 220, for example using a method of operation as is explained below in relative to the flow charts in Figs. 9A-9C. As seen in Fig. 8, the controller 234 may include a microprocessor 280 (which, in fact may include multiple physical and/or virtual processors). According to other embodiments, the controller 234 may include one or more electrical circuits designed to carry out the actions described herein. In fact, the controller 234 may include a microprocessor 280 and other circuits or circuitry. In addition, the controller 234 may include at least one memory 282. The instructions by which the microprocessor 280 is programmed may be stored on the at least one memory 282 associated with the microprocessor 280, which at least one memory 282 may include one or more tangible non-transitory computer readable memories, having computer executable instructions stored thereon, which when executed by the microprocessor 280, may cause the microprocessor 280 to carry out one or more actions as described herein.

As mentioned above, the controller 234 may be coupled (i.e., directly or indirectly connected) to the equipment of the reusable hardware 232, such as the spinning membrane drive 270, the first syringe pump 272, the second syringe pump 274, and the cassette interface 276. The controller 234 may operate each of these devices, each of which may be an assembly of other devices or equipment, to cause the fluid to flow through the fluid circuit 230 associated with the hardware 232, for example to cause fluid to flow from the source container 224, through the spinning membrane 240, and eventually into the container 106.

For example, the controller 234 may be programmed to perform a process or procedure according to a protocol, such as to wash particular cells contained in the fluid within the source container 224, before they are directed to the container 106. The controller 234 may be programmed to perform other actions as well, such as to test the fluid circuit 230, to prime the fluid circuit 230, to rinse parts of the circuit 230 after the wash has been performed, and to add other components to the cell-containing fluid before that fluid is distributed to the container 106.

As is also illustrated in Fig. 8, the controller 234 may be coupled to one or more of the structures described above, for example to receive information (e.g., in the form of signals) from these structures or to provide commands (e.g., in the form of signals) to these structures to control the operation of the structures. As illustrated, the controller 234 may be coupled to at least one input device 284 to receive information from that device and to at least one output device 286 to provide information to that device. The at least one input device 284 may include a number of different devices according to the embodiments described herein. For example, the input device 284 may include a keyboard or keypad by which a user may provide information and/or instructions to the controller 234.

Alternatively, the input device 284 may be a touch screen, such as may be used in conjunction with an output device 286 in the form of a video display. For instance, the illustrated embodiment in Figs. 6 and 7 includes such a touch sensitive display screen 288 mounted to a front panel 290 of a housing 292 of the system 220. The input device may also include a reader or scanner, such as a barcode reader or scanner or an RFID reader. According to still other embodiments, the input device may be in the form of computer equipment that permits the cell processing system including the controller 234 to communicate (whether via wires, cables, etc. or wirelessly) with other processing systems over a local network, or with other cell processing systems or other computer equipment (e.g., a server) over local networks, wide area networks, or the Internet. According to such an embodiment, the input may include an internal transmitter/receiver device.

The controller 234 may also be coupled to the magnetic selector 100, and in particular the magnet carrier 104. The controller 234 may be configured to move the magnet carrier 104 between the first state wherein the magnet carrier 104 is adjacent the floor 108 and the second state wherein the magnet carrier 104 is spaced from the floor 108. In particular, the controller 234 may be coupled to the motor 202 of the linear actuator 186, and may operate the motor 202 to drive the plate 180 between the two states or positions.

According to one embodiment of an automated method of operating the system 220, and of operating the magnetic selector 100 that is incorporated into the system 220, the magnetic selector 100 is used to select target cells using a magnetic field. By "automated" or "automatically," it is meant that the system (and in particular controller 234) can be programmed to carry out the processing steps of a processing method without substantial operator/user involvement. Of course, even in the automated system of the present disclosure, it will be understood that user activity may be involved, including the loading of the disposable fluid circuits and entering processing parameters. Additional manual steps may be required, as well, and there may be opportunities for reconfiguration of certain steps in a process. However, the reusable apparatus can process the biological fluid through the disposable circuit(s) described below without substantial user intervention.

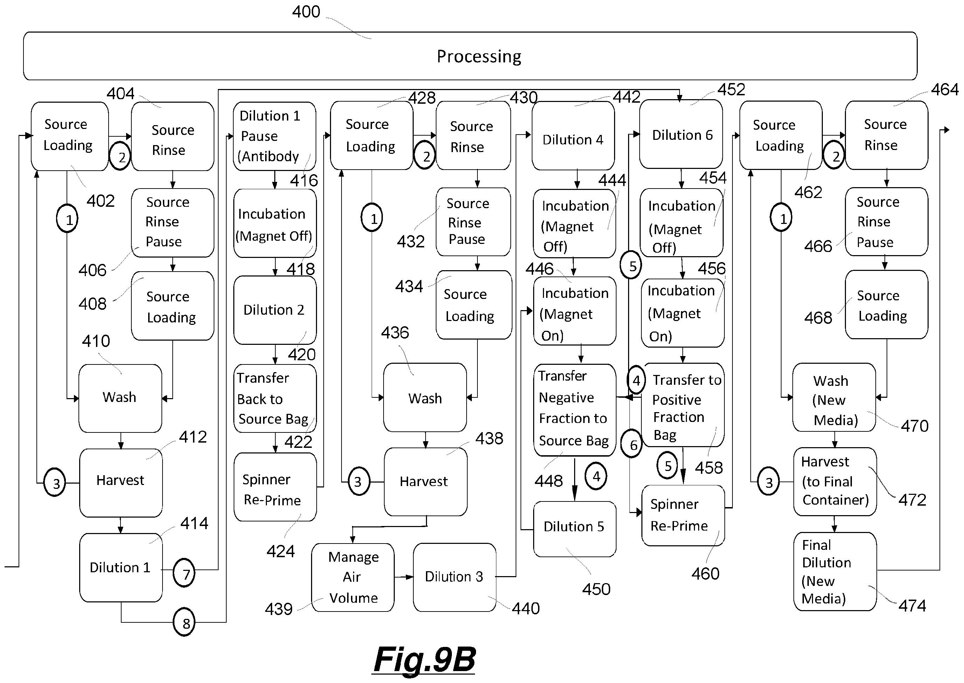

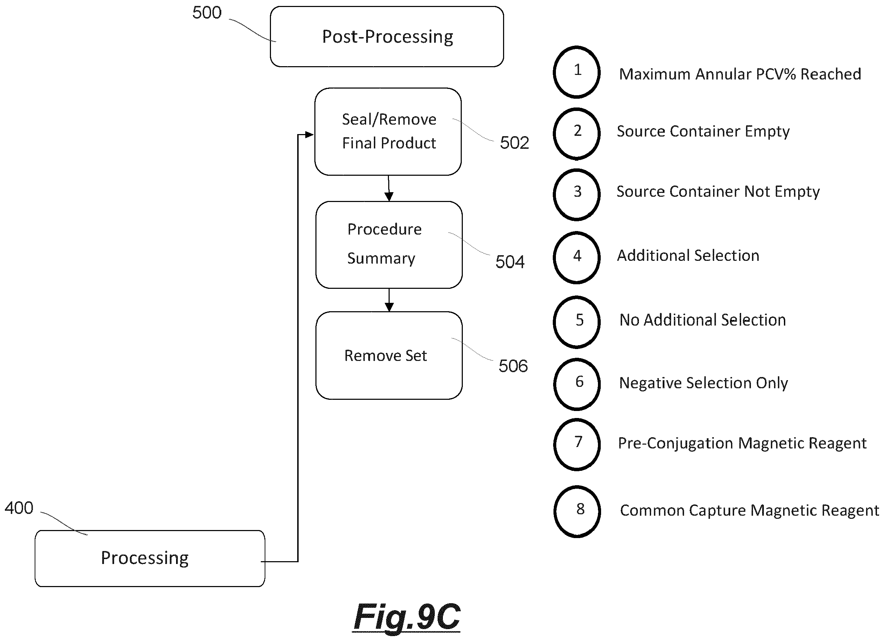

A detailed description of methods of operation of the system begins, in general terms, with the methods including multiple steps within phases or states of Pre-Processing 300, Processing 400, and Post-Processing 500. Flow charts representing some alternatives for the steps in these states can be seen in the figures, such as pre-processing 300 in Fig. 9A, processing 400 in Fig. 9B and post-processing 500 in Fig. 9C.

The user may first activate (e.g., switch on) the hardware 232. The hardware 232 with controller 234 may conduct self-calibration checks, including the checking of the pumps 272, 274, and other components. Similar self-calibration checks may be performed relative to the selector 100 when the user activates the selector 100, or when the user activates the hardware 232.

As shown in Fig. 9A, Pre-Processing 300 starts at block 302 with Protocol Selection. In this step, the fluid processing system 220 displays a list of protocols from which to choose to run a cell processing procedure. The related protocol parameters for this step may include a respective Protocol ID and Protocol Description. Next, at block 304 the method includes Procedure Setup. During Procedure setup for a selected protocol, the system controller 234 prompts the user to enter information, which for example may include identification, consumables, and source composition that are pertinent to the specific selected procedure to be executed and which may be entered via the input 284. For this step 304, the related protocol parameters may more specifically include pertinent information including: a required Procedure ID, User ID and Source ID, as well, for example identifying the Target Cell, Cell Component, Component Retention Flag (for Secondary Wash and Post-Selection Wash), Primary Set Reference Number, Primary Set Confirm Reference Configured, Solution 1 Name, Solution 1 Reference Number, Solution 2 Name, Solution 2 Reference Number, Solution 3 Name, Solution 3 Reference Number, Solution 4 Name, Solution 4 Reference Number, and Final Product Bag Max Fill Volume (mL). Also, the hardware 232 may prompt the user to enter or modify process parameters using the input 284, including by way of example and not by way of limitation the amount of cell suspension to be processed, the number of cycles to take place, etc.

Pre-Processing 300 next includes at block 306 a step to Install Set. This involves the controller 234 of the fluid processing system 220 prompting the user to install the disposable fluid circuit 230 (also referred to as the disposable kit, kit or cassette) onto the hardware 232 of the fluid processing system 220. During this step, the fluid processing system 220 will load and seal the fluid circuit 230 into place. Once the kit is installed, at block 308 Installation Checks are performed. In this step, the system checks various parts of the disposable kit 230 for correct installation and confirms the kit integrity.

Pre-Processing 300 continues at block 310 with Attach Solution 1, wherein the system 220 prompts the user to attach Solutions 1 to the disposable kit 230. Solution 1 may be a separation buffer, which may be held by a container, such as container 248, and the related protocol parameter for this step may include the Solution 1 connected volume. When connecting the solutions, the connections, such as of tubing to a container, may be made via a spike connector, sterile connection, or other suitable means of connection.

This is followed at block 312 by the step of Attach Solution 2, wherein the system 220 prompts the user to attach Solution 2 to the disposable kit 230. Solution 2 may be for example a release buffer or wash medium, such as saline solution, which may be held by a container, such as container 250. The related protocol parameter for this step may include the Solution 2 connected volume.

The next step shown in Fig. 9A is at block 314 with the step Attach Solution 3. In this step, the system 220 prompts the user to attach Solution 3 to the disposable kit 230. Solution 3 may be a magnetic reagent or ferrofluid (FF) that contains magnetic particles, which may be held by a container, such as container 246. The related protocol parameters for this step may include the Solution 3 connection point and Solution 3 connected volume. The system 220 also can be configured in an alternative manner to attach Solution 3 just prior to Solution 3 addition that would occur mid-procedure. An optional step at block 316 includes Attach Solution 4. If applicable, the system 220 prompts the user to attach Solution 4 to the disposable kit 230. Solution 4 may be new media held in a further container, and the related protocol parameters for this step may include the Solution 4 connection point and Solution 4 connected volume. The system 220 also may be configured in an alternative manner to attach Solution 4 just prior to Solution 4 addition that would occur mid-procedure.

Once the user has connected the appropriate Solutions, the Pre-Processing 300 continues at block 318 with Solution Prime. The controller 234 of the fluid processing system 220 primes various parts of the fluid circuit or disposable kit 230 to (1) check for correct installation of the tubing lines and (2) clear tubing lines and the spinning membrane separator 240 of air. In an example embodiment, the fluid circuit 230 may be primed with saline, although other biocompatible aqueous solutions may be used.

The next step at block 320 is Attach Source, wherein the controller 234 of the system 220 prompts the user to attach the Source product to the kit 230. The source product may be biological fluids/cells, which may have been recently obtained via apheresis (or leukapheresis), refrigerated overnight or previously cryopreserved material, etc., and may be held for example by source container 224. This is followed at block 322 by Source Prime, wherein the system primes the tubing line leading to the Source product to clear the tubing line of air. This state optionally also can be used to pre-dilute the Source product, if desired. The related protocol parameters for this step may include the Source prime volume (mL) and Source Prime flow rate (mL/min). At this stage, the method may include block 324 for a Source Prime Pause, wherein the system 220 may be configured to pause at the completion of Source Prime step 322 to agitate or mix the Source product container before transitioning to the next state. As such, the related protocol parameters for this step may include Post Prime Pause Configured and Post-Prime Pause Text.

Having completed Pre-Processing 300, the method then enters the state of Processing 400, as seen in Fig. 9B. Fig. 9B includes circled, numbered notes 1 to 8, which correspond to a key shown in Fig. 9C. Processing 400 starts at block 402 with Source Loading (for a Primary Wash cycle). In this step, Source product is drawn into the spinning membrane separator 240, such as through a port on the Source product container 224 and via tubing and use of the first syringe 242 associated with syringe pump 272. Thus, the biological fluid/cells is/are transferred from Source container 224 through the kit 230 to its spinning membrane separator 240 via operation of one or more syringe pumps 272, 274. In a similar fashion, the wash medium may be delivered from its container 250 through the fluid circuit 230 to the spinning membrane separator 240.

During the Primary Wash, the cells within the Source product accumulate within the annular space of the spinning membrane separator 240 until either (1) the Source container is empty (identified in Fig. 9B by circled key 2: Source Container Empty) or (2) the number of cells within the spinning membrane separator 240 reach a pre-configured capacity (identified in Fig. 9B by circled key 1: Maximum Annular PCV% Reached). As cells are loaded into the spinning membrane separator 240, supernatant (filtrate) crosses the membrane and is drawn into first syringe 242. It will be appreciated that the biological cells may be collected in an in-process container while supernatant is separated and removed to a waste container 246.

The related protocol parameters for this step may include spinning membrane separator Loading Revolution Rate (RPM), Source Inlet Flow Rate (mL/min), and Maximum Annular packed-cell volume (PCV) (%).

The next processing step at block 404 is Source Rinse 404 (for Primary Wash). When the Source container 224 is empty, Solution 1 is transferred from container 248 to the Source container 224 to recover any residual cells left in the container and tubing line. The related protocol parameters for this step may include Source Rinse Configured, and Source Rinse Volume (mL). It will be appreciated that for each of the states discussed below, the solution to be used for each step may be configurable by the end user and will depend on the application and the user's processing needs.

This is followed at block 406 by Source Rinse Pause (for Primary Wash). The system 220 then returns to a Source Loading state, at block 408, to process the rinse solution with the residual cells. However, the system 220 can be configured to include at block 406 a Source Rinse Pause after Solution 1 is transferred to the Source container 224, to allow the user to mix the rinse solution in the Source container 224 to capture any residual cells trapped in the container. The text displayed on the screen 288 is configurable to permit the addition of this step. The related protocol parameters for this step may include Source Rinse Pause Configured and Source Rinse Pause Text. If the Source Rinse Pause is utilized, then the system 220 returns to the block 408 step of Source Loading to process the rinse solution with the residual cells.

The Processing 400 continues with Wash 410 (for Primary Wash), wherein with the cells suspended within the annular space of the spinning membrane separator 240, Solution 1 is drawn into the spinner of the separator 240 using the first syringe 242. Residual supernatant and wash buffer cross the spinning membrane 240 and are drawn into the first syringe 242. The related protocol parameters for this step may include Primary Spinner Wash Configured, Primary Wash Solution, Primary Wash Spinner Wash Volume (mL) and Primary Wash Spinner Wash Flow Rate (mL/min). It will be appreciated that this step may be repeated with an additional wash solution. For example, when processing fresh apheresis material for selection, platelets become activated with washing Solution 2. In that instance, the suspension is first washed with Solution 1 to remove platelets to the filtrate, which may then be immediately followed by a wash with Solution 2 to resuspend the cells in the media required for the subsequent steps of processing.

This is followed at block 412 by Harvest (for Primary Wash), wherein the cells within the annular space of the spinner of the spinning membrane separator 240 are drawn out of the spinner with Solution 1 using the second syringe 244 and are transferred to the Selection container, such as container 106 in the example shown, or to an alternative Selection container. If the Source container 224 is not empty, the system 220 will return to the Source Loading (for Primary Wash) state (as identified in Fig. 9B by circle key 3: Source Container Not Empty). If the Source container 224 is empty, the system 220 will transition at block 414 to a Dilution 1 state or step. The related protocol parameters for this Harvest (for Primary Wash) step may include Harvest Volume (mL), and Harvest Flow Rate (mL/min). In the block 414 Dilution 1 step, the system 220 dilutes the cell suspension in the Selection container immediately after harvesting the cells from the spinning membrane separator 240 with Solution 1. The related protocol parameters for this Dilution 1 step may include Dilution 1 Volume (mL), and Dilution 1 Flow Rate (mL/min).

At 414, the system may prompt the user to attach a Negative Fraction Holding Container to the disposable kit 230 at 224 or to open the fluid pathway to a preconnected Negative Fraction Holding Container. Following the Dilution 1 at block 414, the procedure may proceed to block 452 for the Dilution 6 step (as identified in Fig. 9B by circle key 7: Pre-Conjugation Magnetic Reagent) to undergo a Positive Selection (discussed below), or (as identified by circle key 8: Common Capture Magnetic Reagent) the process may proceed to the next step at block 416 which is Dilution 1 Pause. In Dilution 1 Pause at block 416, the system 220 may be configured to pause at the completion of the Dilution 1 state. This is the point in the procedure at which the user may manually add antibody directly to the Selection container. For example, the user may manually inject a monoclonal antibody (mAb) solution into the solution containing the target cells, for example, an introducer container (not shown) may be attached to the container either prior to the procedure or in a sterile manner during the procedure and the mAb solution may be injected into the container from the introducer container. According to other embodiments the monoclonal antibody may be introduced automatically into the container. The related protocol parameters for this Dilution 1 Pause may include Dilution 1 Pause Configured, Dilution 1 Pause Text, Dilution 1 Sample Volume (mL) and Antibody Volume (mL).

In turn, this may be followed at block 418 by Incubation. The Incubation is antibody incubation wherein the cell suspension in the Selection container 106 incubates with the added antibodies. The duration of the incubation and agitation are configurable by the user via the at least one input device 284. At this state, the magnet carrier 104 of the magnetic selector 100 is disengaged by being in the second state, spaced from the floor 108, and the related protocol parameters may include Incubation Time, Incubation Agitation Enabled, Incubation Agitation Rate, and Incubation Agitation Angle.

At block 420 Dilution 2, the system may further dilute the cell suspension with Solution 1 to target a specific cell concentration or volume for a Secondary Wash. This is followed at block 422 by Transfer Back To Source Bag, where the cell suspension is transferred from the Selection container back to the original Source container (or a newly connected container) using the second syringe 244. At block 424 Spinner Re-Prime, the system re-primes segments of the disposable kit 230 between the Source container 224 and the second syringe 244 to clear the air. The related protocol parameters at this state may include Post-Antibody Spinner Reprime Solution, Post-Antibody Source Prime Volume (mL), and Post-Antibody Source Prime Flow Rate (mL/min). This may be followed optionally by a Spinner Re-Prime Pause, wherein the system 220 may be configured to pause at the completion of the Spinner Re-Prime to mix the cell suspension in the Source container 224 before transitioning to the next state. If reconfiguring to utilize the Spinner Re-Prime Pause, the related protocol parameters may include Post-Antibody Source Prime Pause Configured, and Post-Antibody Source Prime Pause Text.

As shown in the example in Fig. 9B, the Processing 400 at block 428 may include a further Source Loading (for a Secondary Wash cycle). Accordingly, at block 428 the cell suspension is drawn into the spinner of the spinning membrane separator 240 through the port on the Source product container 224 using the first syringe 242. Similar to the Primary Wash, the cells accumulate within the annular space of the spinner until either (1) the source container is empty (identified in Fig. 9B by circled key 2: Source Container Empty) or (2) the number of cells within the spinner reach a pre-configured capacity (identified in Fig. 9B by circled key 1: Maximum Annular PCV% Reached). As cells are loaded into the spinner, supernatant (including excess antibodies) crosses the membrane of the spinning membrane separator 240 and is drawn into the first syringe 242. The related protocol parameters may include Spinner Loading Revolution Rate (RPM), Source Inlet Flow Rate (mL/min), and Maximum Annular PCV (%).

The next processing step at block 430 is Source Rinse 404 (for Secondary Wash). When the Source container 224 is empty, Solution 2 is transferred from container 250 to the Source container 224 to recover any residual cells left in the container and tubing line. The related protocol parameters for this step may include Source Rinse Configured, and Source Rinse Volume (mL).

The system 220 then returns to a Source Loading state, at block 434, to process the rinse solution with the residual cells. However, the system 220 can be configured to include at block 432 a Source Rinse Pause. Thus, after Solution 2 is transferred to the Source container 224, the system 220 can be configured to pause to allow the user to mix the rinse solution in the Source container 224 to capture any residual cells trapped in the container. The text displayed on the screen 288 is configurable to permit the addition of this step. The related protocol parameters for this step may include Source Rinse Pause Configured, and Source Rinse Pause Text. If the Source Rinse Pause at block 432 is utilized, then the system 220 returns to the block 434 step of Source Loading to process the rinse solution with the residual cells.

The Processing 400 continues at block 436 with Wash (for Secondary Wash), wherein with the cells suspended within the annular space of the spinner of the spinning membrane separator 240, Solution 2 is drawn into the spinner of the separator 240 using the first syringe 242. Residual supernatant and wash buffer cross the spinning membrane 240 and are drawn into the first syringe 242. The related protocol parameters for this step may include Secondary Spinner Wash Configured, Secondary Wash Spinner Wash Volume (mL) and Secondary Wash Spinner Wash Flow Rate (mL/min).

This is followed at block 438 by Harvest (for Secondary Wash), wherein the cells within the annular space of the spinner of the spinning membrane separator 240 are drawn out of the spinner with Solution 2 using the second syringe 244 and are transferred to the Selection container, such as container 106 in the example shown, or to an alternative Selection container. If the Source container 224 is not empty, the system 220 will return to the Source Loading (for Secondary Wash) state (as identified in Fig. 9B by circle key 3: Source Container Not Empty). The related protocol parameters for the Harvest (for Secondary Wash) step may include Harvest Volume (mL), and Harvest Flow Rate (mL/min). If the Source container 224 is empty, the system 220 will transition at block 439 to Manage Air Volume state. In block 439, the Selection container, such as container 106 or an alternative Selection container, is moved to an angled position, using the hardware discussed herein for otherwise imparting agitation by rocking the magnetic selector 100. With the container inlet raised to be above the majority of the container, the air entrapped in the Selection container is made available at the container inlet. The air then is drawn out of the container by the second syringe pump 274 and vented through the cassette 260 via a cassette filtered vent. This process is repeated until air is detected at the second syringe pump 274 by one of the cassette sensor stations, indicating the removal of the air. At this point, a known volume of air (for example, 50-300mL) is reintroduced into the syringe pump 724 via the cassette filtered vent. Syringe pump 274 then directs the known volume of air into the Selection container 106. This volume of air is used to optimize the recovery of magnetically-labeled cells. Too much air in the selection container would limit the volume of fluid that can be co-contained in the selection container, and thereby limit the cell capacity of a selection. Too little air in the selection container may cause walls of a flexible container to collapse upon each other during draining operations, which can cause magnetically captured cells to be lost to the negative fraction. The related protocol parameters for the Manage Air Volume state include the Selection Container Air Volume (ml).

The system 220 then will transition at block 440 to a Dilution 3 state. In the block 440 Dilution 3 step, the system 220 dilutes the cell suspension in the Selection container after harvesting the cells from the spinner of the spinning membrane separator 240 with Solution 2 and concluding the Manage Air Volume step. The related protocol parameters for this Dilution 3 step may include Dilution 3 Volume (mL), Dilution 3 Solution, and Dilution 3 Flow Rate (mL/min). If the system 220 is optionally configured to have the Solution 3 attached mid-procedure, the system prompts the user to attach Solution 3 to the fluid circuit or disposable kit 230.

The Processing 400 continues at block 442 with Dilution 4, wherein the system 220 may be configured to automatically add Solution 3 containing magnetic particles to the Selection container, such as container 106. Conversely, the magnetic particles may be added manually to the Selection container by the user. The magnetic particles bind to the antibodies added after the Primary Wash at block 416. The related protocol parameters for this Dilution 4 step may include Dilution 4 Configuration (Automated/Manual), Dilution 4 Volume (mL), and Dilution 4 Flow Rate (mL/min).

This may be followed at block 444 by Incubation (Magnet Off). The cell suspension in the Selection container incubates with the magnetic particles. The duration of the incubation and agitation are configurable by the user via the at least one input device 284. At this state, the magnet carrier 104 of the magnetic selector 100 is disengaged by being in the second state, spaced from the floor 108, and the related protocol parameters may include Incubation Time, Incubation Agitation Enabled, Incubation Agitation Rate and Incubation Agitation Angle.

The next step at block 446 is Incubation (Magnet On), which may otherwise be referred to as negative selection incubation with the magnet engaged. The magnet carrier 104 is engaged and moved to the first carrier state wherein the magnets are adjacent the floor 108 and magnetically bound cells are pulled toward the magnets. The duration of the selection and agitation are configurable by the user. The related protocol parameters may include Incubation Time, Incubation Agitation Enabled, Incubation Agitation Rate, and Incubation Agitation Angle.

The Processing 400 continues at block 448 for the step of Transfer Negative Fraction To Negative Fraction Container. In this step, the magnet is still engaged, and the unbound cells that remain in suspension are removed from the Separation container with the second syringe 244. The removed cells are then transferred to a negative fraction holding container in place of the Source container 224. The negative fraction holding container is not shown in Fig. 7, but may be a container that replaces container 224 or is preconnected via a Y-site connection with container 224. The number of negative selections is configurable by the user. There may be no additional negative selection configured (as identified in Fig. 9B by circle key 5: No Additional Selection). Alternatively, if an additional negative selection is configured, the system 220 again dilutes and re-suspends the cells remaining in the Selection container 106 with Solution 2, re-incubates, and repeats the transfer of the negative fraction to the negative fraction holding container. After the final negative selection is completed, the system chases the fluid pathway from the second syringe 244 to the negative fraction holding container to remove any residual cells.

At block 448 for the step of Transfer Negative Fraction To Source Bag, if a Positive Selection is configured to be run as part of the procedure, the system then transitions to block 450 for the Dilution 5 state (as identified in Fig. 9B by circle key 4: Additional Selection). If a Positive Selection is not configured (as identified in Fig. 9B by circle key 6: Negative Selection Only), the system 220 may either be complete and prompt the user to seal and remove the product in the selection container 106 or transition to the Positive Fraction Sample Pause state, which would occur between blocks 460 and 462, depending on the Post-Selection Wash Configuration. The related protocol parameters at block 448 may include Number of Negative Selections, Negative Fraction Spinner Rinse Volume (mL), Negative Fraction Air Chase Volume (mL), Positive Selection Configuration, and Post- Selection Wash Configuration.

As noted, Dilution 5 is provided at block 450. With the magnet carrier 104 disengaged in the second state by being spaced from the floor 108 of the magnetic selector 100, the positively selected cells are re-suspended and diluted with Solution 1. The contents of Solution 1 act on the magnetic reagent, thereby releasing the cells from the magnetic particles or beads. The related protocol parameters may include Dilution 5 Flow Rate (mL/min), Dilution 5 Solution, and Dilution 5 Volume (mL).

There exists an alternative step from block 452 Dilution 6 to block 454 Incubation. The Incubation at block 452 provides release buffer incubation wherein the cell suspension in the Selection container 106 incubates with Solution 1. The duration of the incubation and agitation are configurable by the user via the at least one input device 284. At this state, the magnet carrier 104 is disengaged by being moved to the second state spaced from the floor 108 of the magnetic selector 100. The related protocol parameters may include Incubation Time, Incubation Agitation Enabled, Incubation Agitation Rate, and Incubation Agitation Angle. Use of such an incubation state may be desired, depending on the release time associated with the magnetic reagent or FF.

This is followed at block 456 by further Incubation (Magnet On). This further Incubation provides positive selection incubation via magnetic engagement. The magnet carrier 104 is engaged by being moved to the first state adjacent the floor 108 of the magnetic selector 100. The free magnetic beads are pulled toward the magnets 120 on the magnet carrier 104. The duration of the selection and agitation during this Incubation are configurable by the user via the at least one input device 284. The related protocol parameters may include Incubation Time, Incubation Agitation Enabled, Incubation Agitation Rate, and Incubation Agitation Angle.

The Processing 400 continues at block 458 for the step of Transfer To Positive Fraction Bag. In this step, the magnet is still engaged, and the released cells that remain in suspension are removed from the Separation container with the second syringe 244. If a Post Selection Wash is configured to be run with the procedure, the removed cells are then transferred to a positive fraction holding container in place of the Source container 224. The positive fraction holding container is not shown in Fig. 7, but may be a container that replaces container 224 or is preconnected via a Y-site connection with container 224. If a Post-Selection Wash is not configured for the procedure, the removed cells are transferred to the final product bag, the procedure is complete and the system 220 advances to Post-Processing 500 wherein it prompts the user to seal and remove the final product. The number of positive selections is configurable by the user.

If an additional positive selection is configured, the system 220 dilutes and re-suspends the cells remaining in the Selection container 106 with Solution 1, re-incubates, and repeats the transfer of the positive fraction to the holding container. After the final positive selection is completed (as identified in Fig. 9B by circle key 5: No Additional Selection), the system 220 chases the fluid pathway from the second syringe 244 to the positive fraction holding container (or final product container depending on the Post-Selection Wash configuration) to remove any residual positively selected cells. After the final positive selection, if a Post-Selection Wash is configured, the system 220 transitions to a Target Fraction Sample Pause within block 458, following the transfer of all the selected cells to the positive fraction holding container. The user has the option to sample and update the cell concentration. The related protocol parameters for the Target Fraction Sample Pause state may include Target Cell Pause Configured, Target Cell Pause Text, Target Cell Sample Volume (mL), and Actual Target Cell Concentration.

If a Post Selection Wash is configured, the system prompts the user to attach Solution 4 to the disposable kit 230. The system 220 then advances to block 460 for Spinner Re-Prime. In this step, the system 220 re-primes segments of the fluid circuit or disposable kit 230 between the positive fraction holding container and the second syringe 244 with Solution 4 to clear the air. The related protocol parameters for connecting Solution 4 may include Solution 4 Connected Volume, while the related protocol parameters for Spinner Re-Prime may include Post-Selection Prime Volume (mL), and Post-Selection Source Prime Flow Rate (mL/min). Block 460 may also be configured to include a Post-Selection Source Prime Pause, wherein the system 220 may pause at the completion of the Spinner Re-Prime to mix the cell suspension in the positive fraction holding container before transitioning to the next state. The related protocol parameters for such a Post-Selection Source Prime Pause may include Post-Selection Prime Pause Configured, and Post-Selection Prime Text.

When a final wash is configured Post-Selection, the next state at block 462 is Source Loading. The cell suspension is drawn into the spinner of the spinning membrane separator 240 through the source port using the first syringe 242. The cells accumulate within the annular space of the spinner until either (1) the positive fraction holding container (not shown) is empty (as identified in Fig. 9B by circle key 2: Source Container Empty) in which case the next step is at block 470 Wash (discussed below) or (2) the number of cells within the spinner reach a pre-configured capacity (as identified in Fig. 9B by circle key 1: Maximum Annular PCV% Reached). As cells are loaded into the spinner of the spinning membrane separator 240, supernatant (filtrate) crosses the membrane and is drawn into first syringe 242. The related protocol parameters for such a Post-Selection Source Loading may include Post-Selection Spinner Loading Revolution Rate (RPM), Post-Selection Source Inlet Flow Rate (mL/min), and Post-Selection Maximum Annular PCV (5%).

When the positive fraction holding container (not shown) is empty (as identified in Fig. 9B by circle key 2: Source Container Empty) the method proceeds to block 464 for Source Rinse. This is a Post-Selection Source Rinse, wherein when the positive fraction holding container is empty, Solution 4 (which replaces Solution 1 or Solution 2 at location 248 or 250, depending on the protocol) is transferred at block 460 to the positive faction holding container to recover any residual cells. The system returns to the Post-Selection Source Loading state to process the rinse solution with the residual cells. The related protocol parameters for this Source Rinse step may include Post-Selection Source Rinse Configured, and Post-Selection Source Rinse Volume (mL).

At block 466 the system 220 reaches a Source Rinse Pause. This is a Post-Selection Source Rinse Pause wherein after Solution 4 is transferred to the positive fraction holding container (not shown), the system 220 may be configured to pause to allow the user to mix the rinse solution in the container to capture any residual cells trapped in the container. The text displayed on the display screen 288 is configurable. The related protocol parameters for this step may include Post-Selection Source Rinse Pause Configured, and Post-Selection Source Rinse Pause Text. This continues at block 468 Source Loading, in preparation for the further Wash.

The next step is at block 470 Wash (New Media), whether proceeding from block 462 due to the number of cells within the spinner reaching a pre-configured capacity (as identified in Fig. 9B by circle key 1: Maximum Annular PCV% Reached), or progressing from the further Rinse steps and Source Loading at block 468. With the cells suspended within the annular space of the spinner of the spinning membrane separator 240, Solution 4 is drawn into the spinner using the first syringe 242. Residual supernatant and wash buffer cross the membrane and are drawn into the first syringe 242. The related protocol parameters for this step may include Post-Selection Spinner Wash Configured, Post-Selection Wash Volume (mL), and Post-Selection Wash Flow Rate (mL/min).

The Processing 400 continues at block 472 for Harvest (to Final Container). In this Harvest step, the cells within the annular space of the spinner of the spinning membrane separator 240 are drawn out of the spinner with Solution 4 using the second syringe 244 and transferred to the final product container (not shown), which would be user-connected container (either bag, culture vessel, or other container) connected to fluid pathway 226. If the positive fraction holding container (not shown) is not empty (as identified in Fig. 9B by circle key 3: Source Container Not Empty), the system 220 will return to the Source Loading (Post-Selection) step at block 462. If the positive fraction holding container is empty, the system 220 will transition to the Final Dilution (New Media) state at block 474. The related protocol parameters for this step may include Harvest Volume (mL), and Harvest Flow Rate (mL/min).

In the further Final Dilution (New Media) step at block 474, the system dilutes the cell suspension in the final product container (not shown) connected to fluid pathway 226 immediately after harvesting the cells from the spinner with Solution 4 to achieve the target final product volume or targe final concentration. The related protocol parameters for this step may include Final Dilution Flow Rate (mL/min), Final Dilution Entry Method, Final Dilution Concentration, Final Dilution Volume (mL), and Final Dilution Air Chase Volume (mL).

After the Final Dilution (New Media) step, the Processing 400 may be configured to have the system 220 include a Residual Rinse. If so configured, the system 220 will add solution back to the Selection container 106 to resuspend the free magnetic beads and any residual cells. The related protocol parameters if including a Residual Rinse step may include Residual Rinse Configured, Residual Rinse Solution, Residual Rinse Volume (mL), and Residual Rinse Flow Rate (mL/min).

The system 220 then transitions to Post-Processing 500, which includes at block 502 Seal/Remove Final Product. At this step, the system 220 prompts to seal and remove the final product container (not shown) attached to fluid pathway 226. This may be followed at block 504 by a Procedure Summary step, wherein the system displays a summary of the procedure, such as on the screen display 288. Finally, at block 506 Remove Set, the system 220 prompts the user to remove the disposable fluid circuit or kit 230 from the fluid processing hardware 232.

According to the above description, portions of the present disclosure may be discussed more generally with a focus on use of the magnetic selector 100. For instance, a magnetic selector 100, whether used independently or as part of a system 220, may be used with a fluid that has been combined with other solutions to prepare target cells for selection.

For example, a monoclonal antibody solution may be introduced to the solution containing the target cells, and incubated for a period of time to allow for interaction between the monoclonal antibodies in the solution and the target cells (which may be white blood cells of a particular phenotype, such as CD34+ peripheral blood stem cells, CD3+/CD28+ T-cell lymphocytes, and CD8+ plasma B-cells, which cells may also be referred to as target cells). The spinning membrane separator 240 may be used to mix the cells and then to wash the cells, removing any unbound monoclonal antibodies.

At this point, a non-specific magnetic particle solution (e.g., a magnetic bead solution or magnetic reagent, such as ferrofluid (FF)) may be introduced to the suspension. For example, an introducer container (not shown) may be attached to the container either prior to the procedure or in a sterile manner during the procedure and the ferrofluid is injected into the container from the introducer container. According to other embodiments, the ferrofluid may be introduced automatically into the container. The contents of the container may be incubated for a period of time to allow for interaction between the ferrofluid and the non-specific end of the monoclonal antibodies, mixed and (optionally) washed to remove any unbound ferrofluid.

Alternatively, the monoclonal antibody and the magnetic particles may be combined prior to introduction into the container 106. In this case, the combination of the monoclonal antibody and the magnetic particles may be incubated with the cells to form the complex in a single set of steps, as opposed to the two related sets of the steps outlined above.

Once the complex has been formed, the fluid may be moved into the container 106. The magnet carrier 104 may be actuated and/or positioned adjacent the container 106 to attract the magnetic particles, and in particular the magnetic particles associated with the target cells, to a particular portion of the container 106 (e.g., the lower section of the container 106 adjacent the floor 108). The container 106 may then be agitated and the negative fractions removed. Additional fluid then may be added to the container to achieve a desired final volume, and the magnet carrier 104 may be moved relative to the floor 108 so that the carrier 104 and the floor 108 are spaced. Once the processing is completed, the controller 234 may prompt the user to sample, seal and remove the container 106.

As mentioned above in the detailed description of one example method of operation, the selector 100 may utilize agitation. To permit the fluid in the container 106 to be agitated, the selector 100 may be mounted on a shaft 294 that depends from the housing 292. The shaft 294 is attached to a motor mounted in the housing 292, which motor may cause the shaft to rotate in opposite directions about an axis of the shaft. As a consequence, the fluid in the container 106 may be agitated in an oscillatory fashion.

The mounting of the selector 100 on the shaft 294 may also permit the container 106 to be disposed at an angle to the horizontal, instead of having the container disposed such that the container is level with the horizontal. For example, the selector 100 may be oriented relative to the horizontal such that the selector 100 is disposed at 30 degrees to the horizontal. Of course, this could be achieved in a standalone version of the selector 100 as well, either by providing a mechanism (such as a pivot) that permits the selector 100 (or the floor surface 114) to be at a variable degree of orientation to the horizontal (or vertical) or a mechanism (such as an inclined plane) that permits the selector 100 to be at a particular degree of orientation to the horizontal (or vertical).

It also would be helpful to provide a brief description of the operation of the magnetic selector 100, which may be aided by reference to a flow chart in Fig. 10 showing a method of operation 600. To begin the magnetic separation or selection of the target cells, block 602 provides a first step of Container Disposed In Selector. In this step, the container 106 is disposed on the floor 108 of the magnetic selector 100. If the container is rigid-walled, the door 110 of the selector 100 may be optional. Assuming the container 106 is flexible, the next step at block 604 is Close Door. Thus, the door 110 is moved from the open state to the closed state at block 604 with the flexible container 106 between the floor surface 114 and the facing door surface 116. The contents of the in-process container may be transferred to the container 106 before or after steps 602, 604.

The method 600 continues to block 606 for the step Carrier First State, wherein the magnet carrier 104 is moved to the first state, adjacent the floor 108, to apply a magnetic field to the fluid in the container 106. The application of the magnetic field causes the target cells associated with the magnetic particles and any unbound magnetic particles to migrate to a portion of the container 106, in particular the portion of the container 106 adjacent the floor 108.

The next step in the method is Incubate at block 608. In this step, the contents of the container 106 are permitted to remain in container 106 for a period of time, with the magnet carrier 104 in the first state. The method may continue to block 610 for an Agitate step, wherein the contents of the container 106 are agitated, which may occur with the magnet carrier 104 in the second state, spaced from the floor 108 of the magnetic selector 100. For example, the shaft 294 may be rotated such that the tray 130 may be alternatively inclined back and forth between a position where the first end 138 is higher than the second end 140 and a position where the second end 140 is higher than the first end 138. The actions of blocks 606-610 may be repeated as multiple cycles over a longer period of time (e.g., several minutes). Once this portion of the method 600 is complete, the next step is Carrier Second State at block 612. In this step, the magnet carrier 104 is moved to the second state, spaced from the floor 108 of the selector 100.

At block 614, the step Add Diluent is performed. Fluid (diluent) may be added to the container 106 from one of the wash containers 248, 250. Once the desired volume is achieved in the container 106, the method advances to block 616 for Carrier First State. In this step, the magnet carrier 104 may be moved to the first state, adjacent the floor 108 of the selector 100. The method then undergoes the step Incubate at block 618, wherein the contents of the container 106 are incubated for a period of time. The next step at block 620 is Remove Fraction, for removal of the negative fraction, which may include fluid, diluent, and other material not attracted to the magnet carrier 104, and therefore, not in the portion of the container 106 most adjacent the floor 108 of the selector 100. The removal of the negative fraction may occur via one of the syringes 242, 244 working with a respective syringe pump 272, 274. Positioning of the container 106 also may be adjusted, for example to position the tray 130 with the end 140 disposed at a lower elevation than the end 138.

After the Remove Fraction step at block 620, the method 600 may proceed to the step Rinse at block 622. The container 106 receives fluid to remove any cells that are not target cells associated with magnetic particles. At least one pump 272, 274 may be operated to add diluent (e.g., wash solution) from a respective container 248, 250 to the container 106. At the same time or shortly thereafter, the magnet carrier 104 is moved to the second state, spaced from the floor 108 of the selector 100, and the container 106 may be agitated. The magnet carrier 104 may be moved to the first state, the contents of the container incubated. The diluent may then be removed from the container 106. The actions at block 622 may be repeated for multiple cycles, as may be desired.

Once the desired number of cycles has been completed, the method 600 continues to block 624 for Carrier Second State. In this step, the magnet carrier 104 is moved to the second state, spaced from the floor 108 of the separator 100. Add Diluent is the next step, at block 626, wherein the volume of the container 106 is brought to its final amount by operating syringe pumps 272, 274 to transfer fluid from one or more of the containers 248, 250 to the container 106. The container 106 optionally may also be further agitated at block 628 Agitate, by varying the inclination of the container 106 either at the same time as fluid is transferred to the container 106 or shortly thereafter.

The door 110 of the selector 100 then may be moved to the open state, and the container 106 may be sealed and removed from the magnetic selector 100. According to certain embodiments, the positive fraction magnetically selected and retained in the container 106 may be the desired product. According to other embodiments, the positive fraction within the container 106 may be transferred from the container 106 into one or more additional containers, which containers may include a volume appropriate for administration or delivery to a patient and which containers may be delivery containers, such as syringes.

Thus, an improved system and methods for applying a magnetic field to a container having disposed therein a fluid including cells is discussed herein, and the magnetic selector may be utilized independently or incorporated into a system and method for processing a biological fluid. The description provided above, and the other aspects provided below, are intended for illustrative purposes, and are not intended to limit the scope of the disclosure to any particular method, system, apparatus, or device described herein.

### Other Aspects

Aspect 1. A method of operating a magnetic selector, the magnetic selector comprising a housing including a floor having a floor surface, a magnet carrier disposed on an opposite side of the floor from the floor surface and having at least one magnet disposed thereon, the magnet carrier being moveable relative to the floor between a first state wherein the magnet carrier is adjacent the floor and a second state wherein the magnet carrier is spaced from the floor, the method comprising: disposing a container on the floor surface; moving the magnet carrier to the first state to apply a magnetic field to a fluid in the container; moving the magnet carrier to the second state from the first state to disengage the magnetic field from the container; and removing the container.

Aspect 2. The method of operating the magnetic selector according to aspect 1, further comprising agitating the fluid in the container while the container is disposed on the floor surface.

Aspect 3. The method of operating the magnetic selector according to any one of aspects 1 or 2, further comprising agitating the fluid in the container after moving the magnet carrier to the second state.

Aspect 4. The method of operating the magnetic selector according to any one of aspects 1 or 2, further comprising: moving the magnet carrier to the first state to apply a magnetic field to the fluid in the container after agitating the fluid in the container; moving the magnet carrier to the second state from the first state to disengage the magnetic field from the container; and adding diluent to the container after moving the magnet carrier to the second state.

Aspect 5. The method of operating the magnetic selector according to any one of aspects 1 to 3, further comprising: moving the magnet carrier to the first state to apply a magnetic field to the fluid in the container after adding the diluent to the container; and removing a portion of the fluid and diluent from the container.

Aspect 6. The method of operating the magnetic selector according to any one of aspects 1 to 5, further comprising adding diluent to the container after removing a portion of the fluid and diluent from the container.

Aspect 7. The method of operating the magnetic selector according to any one of aspects 1 to 6, further comprising adding a magnetic particle to the fluid in the container prior to moving the magnet carrier to the first state to apply a magnetic field to the fluid in the container, the magnetic particle forming a complex with a target cell in the fluid.

Aspect 8. The method of operating the magnetic selector according to any one of aspects 1 to 7, wherein the housing of the magnetic selector further comprises a door moveable relative to the floor between an open state and a closed state, the door having a facing door surface being a fixed distance apart from the floor surface in the closed state and defining a space therebetween, the method further including moving the door from the open state to the closed state after disposing the container on the floor surface and moving the door to the open state and removing the container after-moving the magnet carrier to the second state from the first state to disengage the magnetic field from the container.

Aspect 9. The method of operating the magnetic selector according to any one of aspects 1 to 8, wherein the container is flexible or rigid-walled.

Aspect 10. The method of operating the magnetic selector according to aspect 8, wherein the container is flexible and conforms to the space between the floor surface and the facing door surface when the door is in the closed state.

Aspect 11. A method of operating a biological fluid processing system incorporating a magnetic selector, the system having a magnetic selector comprising a housing including a floor having a floor surface, a magnet carrier disposed on an opposite side of the floor from the floor surface of the selector and having at least one magnet disposed thereon, the magnet carrier being moveable relative to the floor between a first state wherein the magnet carrier is adjacent the floor and a second state wherein the magnet carrier is spaced from the floor, the method comprising: disposing a container on the floor surface; adding a fluid having magnetic particles to a fluid in the container so as to form a complex with target cells in the fluid; moving the magnet carrier to the first state to apply a magnetic field to the fluid in the container; moving the magnet carrier to the second state from the first state to disengage the magnetic field from the container; and removing the container.

Aspect 12. The method of operating the biological fluid separation system incorporating the magnetic selector according to aspect 11, further comprising incubating the fluid in the container after adding the fluid having magnetic particles and moving the magnet carrier to the first state.

Aspect 13. The method of operating the biological fluid separation system incorporating the magnetic selector according to any one of aspects 11 or 12, further comprising agitating the fluid in the container while the container is disposed on the floor surface.

Aspect 14. The method of operating the biological fluid separation system incorporating the magnetic selector according to any one of aspects 11 to 13, further comprising agitating the fluid in the container after moving the magnet carrier to the second state.

Aspect 15. The method of operating the biological fluid separation system incorporating the magnetic selector according to any one of aspects 11 to 14, further comprising adding diluent to the container after moving the magnet carrier to the second state.

Aspect 16. The method of operating the biological fluid separation system incorporating the magnetic selector according to any one of aspects 11 to 14, further comprising moving the magnet carrier to the first state to apply a magnetic field to the fluid in the container after adding the diluent to the container; and removing the negative fraction unbound cells from the container.

Aspect 17. The method of operating the biological fluid separation system incorporating the magnetic selector according to aspect 16, further comprising moving the magnet carrier to the second state after removing the negative fraction unbound cells from the container to achieve a selected volume.

Aspect 18. The method of operating the biological fluid separation system incorporating the magnetic selector according to any one of aspects 11 to 17, wherein the housing of the magnetic selector further comprises a door moveable relative to the floor between an open state and a closed state, the door having a facing door surface being a fixed distance apart from the floor surface in the closed state and defining a space therebetween, the method further including moving the door from the open state to the closed state after disposing the container on the floor surface and moving the door to the open state and removing the container after-moving the magnet carrier to the second state from the first state to disengage the magnetic field from the container.

Aspect 19. The method of operating the biological fluid separation system incorporating the magnetic selector according to any one of aspects 11 to 18, wherein the container is flexible and conforms to the space between the floor surface and the facing door surface when the door is in the closed state.

Aspect 20. The method of operating the biological fluid separation system incorporating the magnetic selector according to any one of aspects 11 to 19, wherein the system further comprises at least one pump on a fluid processor configured to transfer into and out of the container, and at least one controller coupled to the fluid processor, the magnetic selector, and the at least one pump.

Aspect 21. A method of operating a fluid processing system, wherein the system comprises a fluid processor that operates on a disposable fluid circuit that is connectable to a source container filled with a biological fluid and configured to separate the biological fluid from the source container into at least two volumes of material, a container connected to the fluid processor along a fluid pathway, a magnetic selector comprising a housing configured to receive the container and a magnet carrier, the housing including a floor having a floor surface upon which the container is disposed, and the magnet carrier being disposed in a space on an opposite side of the floor from the floor surface, the method comprising:
pre-processing, processing, and post-processing states;
the pre-processing state further comprising selection of a procedure protocol, procedure setup, installation of the disposable fluid circuit on the fluid processor and attaching the source container and at least one solution container to the fluid processor;
the processing state further comprising introduction of the biologic fluid in the source container to the disposable fluid circuit, washing, harvesting and dilution of the of the biologic fluid, incubation with magnetic particles to perform negative selection incubation, moving the magnet carrier between a first state adjacent the floor to subject the container to a magnetic field and a second state spaced from the floor to remove the magnetic field, and removal of negative fraction comprising unbound cells that remain in suspension in the container;
the post-processing state further comprising sealing the container and removing the final product, and removing the disposable fluid circuit.

Aspect 22. The method of operating the fluid processing system according to aspect 21, wherein the processing state further performs positive selection incubation and removal of positive fraction comprising released cells that remain in suspension while the magnet carrier remains in the first state.

Aspect 23. The method of operating the fluid processing system according to claims 21 or 22, wherein the fluid processor further comprises hardware and a controller configured to operate the hardware while the disposable fluid circuit is installed on the hardware and is connected to the source container and the container disposed on the floor of the magnetic selector.

## Claims

1. A method of operating a magnetic selector, the magnetic selector comprising a housing including a floor having a floor surface, a magnet carrier disposed on an opposite side of the floor from the floor surface and having at least one magnet disposed thereon, the magnet carrier being moveable relative to the floor between a first state wherein the magnet carrier is adjacent the floor and a second state wherein the magnet carrier is spaced from the floor, the method comprising:
disposing a container on the floor surface;
moving the magnet carrier to the first state to apply a magnetic field to a fluid in the container;
moving the magnet carrier to the second state from the first state to disengage the magnetic field from the container; and
removing the container.

2. The method of operating the magnetic selector according to claim 1, further comprising agitating the fluid in the container while the container is disposed on the floor surface.

3. The method of operating the magnetic selector according to claim 1, further comprising: moving the magnet carrier to the first state to apply a magnetic field to the fluid in the container after agitating the fluid in the container; moving the magnet carrier to the second state from the first state to disengage the magnetic field from the container; and adding diluent to the container after moving the magnet carrier to the second state.

4. The method of operating the magnetic selector according to claim 1, further comprising adding a magnetic particle to the fluid in the container prior to moving the magnet carrier to the first state to apply a magnetic field to the fluid in the container, the magnetic particle forming a complex with a target cell in the fluid.

5. The method of operating the magnetic selector according to claim 1, wherein the housing of the magnetic selector further comprises a door moveable relative to the floor between an open state and a closed state, the door having a facing door surface being a fixed distance apart from the floor surface in the closed state and defining a space therebetween, the method further including moving the door from the open state to the closed state after disposing the container on the floor surface and moving the door to the open state and removing the container after moving the magnet carrier to the second state from the first state to disengage the magnetic field from the container.

6. The method of operating the magnetic selector according to claim 1, wherein the container is flexible or rigid-walled.

7. A method of operating a biological fluid separation system incorporating the method of operating a magnetic selector of claim 1, the method comprising:
disposing a container on the floor surface;
adding a fluid having magnetic particles to a fluid in the container so as to form a complex with target cells in the fluid;
moving the magnet carrier to the first state to apply a magnetic field to the fluid in the container;
moving the magnet carrier to the second state from the first state to disengage the magnetic field from the container; and
removing the container.

8. The method of operating the biological fluid separation system incorporating the magnetic selector according to claim 7, further comprising incubating the fluid in the container after adding the fluid having magnetic particles and moving the magnet carrier to the first state.

9. The method of operating the biological fluid separation system incorporating the magnetic selector according to claim 7, further comprising agitating the fluid in the container while the container is disposed on the floor surface.

10. The method of operating the biological fluid separation system incorporating the magnetic selector according to claim 7, further comprising moving the magnet carrier to the first state to apply a magnetic field to the fluid in the container after adding the diluent to the container; and removing the negative fraction unbound cells from the container.

11. The method of operating the biological fluid separation system incorporating the magnetic selector according to claim 7, wherein the housing of the magnetic selector further comprises a door moveable relative to the floor between an open state and a closed state, the door having a facing door surface being a fixed distance apart from the floor surface in the closed state and defining a space therebetween, the method further including moving the door from the open state to the closed state after disposing the container on the floor surface and moving the door to the open state and removing the container after-moving the magnet carrier to the second state from the first state to disengage the magnetic field from the container.

12. The method of operating the biological fluid separation system incorporating the magnetic selector according to claim 7, wherein the system further comprises at least one pump on a fluid processor configured to transfer fluid into and out of the container, and at least one controller coupled to the fluid processor, the magnetic selector, and the at least one pump.

13. A method of operating a fluid processing system incorporating the method of operating a magnetic selector of claim 1, wherein the system comprises a fluid processor that operates on a disposable fluid circuit that is connectable to a source container filled with a biological fluid and configured to separate the biological fluid from the source container into at least two volumes of material, a container connected to the fluid processor along a fluid pathway, the method comprising:
pre-processing, processing, and post-processing states;
the pre-processing state further comprising selection of a procedure protocol, procedure setup, installation of the disposable fluid circuit on the fluid processor and attaching the source container and at least one solution container to the fluid processor;
the processing state further comprising introduction of the biologic fluid in the source container to the disposable fluid circuit, washing, harvesting and dilution of the of the biologic fluid, incubation with magnetic particles to perform negative selection incubation, moving the magnet carrier between a first state adjacent the floor to subject the container to a magnetic field and a second state spaced from the floor to remove the magnetic field, and removal of negative fraction comprising unbound cells that remain in suspension in the container;
the post-processing state further comprising sealing the container and removing the final product, and removing the disposable fluid circuit.

14. The method of operating the fluid processing system according to claim 13, wherein the processing state further performs positive selection incubation and removal of positive fraction comprising released cells that remain in suspension while the magnet carrier remains in the first state.

15. The method of operating the fluid processing system according to claim 13, wherein the fluid processor further comprises hardware and a controller configured to operate the hardware while the disposable fluid circuit is installed on the hardware and is connected to the source container and the container disposed on the floor of the magnetic selector.
